(19) Europäisches Patentamt
European Patent Office
Office européen des brevets

(11) Numéro de publication: **0 248 746 B1**

# (12) FASCICULE DE BREVET EUROPEEN

(45) Date de publication du fascicule du brevet: **25.04.90**

(51) Int. Cl.⁴: **C07C 209/00**, C07C 209/32

(21) Numéro de dépôt: **87420138.7**

(22) Date de dépôt: **20.05.87**

(54) **Procédé de préparation de 4-fluoroanilines à partir de 4-halogenonitrobenzenes.**

(30) Priorité: **29.05.86 FR 8607916**

(43) Date de publication de la demande: **09.12.87 Bulletin 87/50**

(45) Mention de la délivrance du brevet: **25.04.90 Bulletin 90/17**

(84) Etats contractants désignés: **AT BE CH DE ES FR GB GR IT LI LU NL SE**

(56) Documents cités:
**US-A- 3 900 519**
**US-A- 4 140 719**
**US-A- 4 582 935**

(73) Titulaire: **RHONE-POULENC CHIMIE, 25, quai Paul Doumer, F-92408 Courbevoie Cédex(FR)**

(72) Inventeur: **Desbois, Michel, 526, Chemin du Bois, F-69140 Rillieux la Pape(FR)**

(74) Mandataire: **Le Pennec, Magali et al, RHONE-POULENC INTERSERVICES Service Brevets Chimie 25, Quai Paul Doumer, F-92408 Courbevoie Cédex(FR)**

EP 0 248 746 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne un procédé de préparation de 4-fluoroanilines à partir de 4-halogénonitrobenzènes. Plus particulièrement, il s'agit d'une hydrogénation fluorante catalytique de 4-halogénonitrobenzènes.

Dans l'état de la technique on connait divers procédés d'obtention des 4-fluoroanilines qu'on peut classer en deux grandes catégories:

1. Les procédés classiques, en plusieurs étapes, comme la fluoration par la diazotation d'un aniline en milieu HF suivie de nitration, puis de réduction du 4-fluoronitrobenzène obtenu [G. SHIEMAN et R. PILLARSKI, Ber. 62, 3035 (1925)] ; le déplacement du chlore par le fluor avec l'action de KF sur des 4-chloronitrobenzènes suivi de réduction [G.C. FINGER et C.W. KRUSE, J.A.C.S., 78, 6037 (1956)], ou encore la nitration de 3-chlorofluorobenzène suivi de réduction.

Les inconvénients présentés par ces procédés sont notamment la multiplicité des étapes, la sélectivité médiocre, les conditions sévères et les rendements limités.

2. Les procédés en une seule étape, notamment comme décrit dans les brevets US 2 884 458, US 3 639 482, US 3 558 707, US 3 580 951, US 3 900 519 et US 3 910 958 où les inconvénients sont soit la faible sélectivité envers les 4-fluoroanilines substituées, soit la difficulté de séparation de la 4-fluoroaniline obtenue des produits de départ.

En particulier le brevet US 3 900 519 décrit un procédé de préparation de 4-fluoroanilines éventuellement substituées par réaction de 4-halogénonitrobenzènes avec de l'acide fluorhydrique en présence d'un agent de désoxygénation. Comme exemples d'agents de désoxygénation sont mentionnés le phosphore, le soufre, les trihalogénures de phosphore, les halogénures de soufre à valence plus basse que 6, les halogénures d'arylphosphore, les halogénures d'aryle sulphényle et les triarylphosphines. Ces produits, toxiques et/ou polluants, de manipulation parfois délicate, sont utilisés en quantité importante et sont consommés pendant la réaction, ils ne sont pas recyclables.

Le mélange des 4-halogénonitrobenzènes avec lesdits agents de désoxygénation est très exothermique, et exige donc, un équipement spécifique de refroidissement. En outre, les rendements ne sont pas très élevés par rapport aux conditions employées, par exemple 6 heures à 150°C sous une pression d'au moins 20 bars.

Même en combinant ce dernier brevet, U.S 3 900 519, avec le brevet U.S 4 140 719 qui correspond au premier type de procédés décrits encore appellé procédés classiques, il n'était pas évident de ramplacer l'agent de déoxygénation par un catalyseur d'hydrogénation du type de ceux décrits dans les procédés classiques. En effet, dans ces procédés le catalyseur d'hydrogénation est utilisé en suspension dans le dérivé nitré en présence d'un agent basique tel qu'une amine tertiaire ou l'acétate de sodium, il n'était pas évident de le transférer dans un milieu superacide comme l'acide fluorhydrique. Dans le cas extrême où ce transfert aurait été envisageable il n'était pas évident de prévoir que seul l'halogéne situé en position para du groupe nitro subirait, un échange avec les atomes de fluor du milieu réactionnel.

Il a donc été mis au point un procédé pour la préparation de 4-fluoroanilines à partir de 4-halogénonitrobenzènes qui, sans présenter les inconvénients de l'art antérieur, donne de façon inattendue un haut rendement.

L'invention a pour object un procédé de préparation de 4-fluoroanilines à partir de 4-halogénonitrobenzénes dont l'halogéne en position de n'est pas le fluor caractérisé en ce que l'on fait réagir au moins un 4-halogénonitrobenzène dont l'halogéne en position de n'est pas le fluor avec de l'hydrogène dans l'acide fluorhydrique solvant, en présence d'au moins un catalyseur à base d'un métal choisi parmi le ruthénium, le rhodium, le palladium, l'osmium, l'irridium et le platine.

Sont particulièrement concernés par l'invention les 4-halogénonitrobenzènes de formule générale:

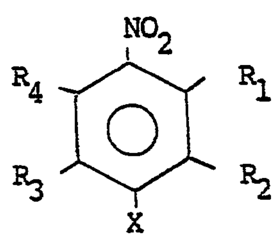

où $R_1$, $R_2$, $R_3$ et $R_4$ sont des substituants identiques ou différents représentant soit l'hydrogène soit des radicaux inertes dans les conditions réactionnelles. Ils sont choisis de préférence parmi les radicaux hydrogène, halogéno, alkyle, perhalogénoalkyle et perhalogénoalcoxy, avantageusement, les radicaux alkyle et alcoxy possèdent de 1 à 4 atomes de carbone, le radical perhalogénoalkyle est un radical perfluoroalkyle et le radical perhalogénoalcoxy est un radical perfluoroalcoxy. Encore plus préférentiellement, le radical perfluoroalkyle est le groupe trifluorométhyle et le radical perfluoroalcoxy est le groupe trifluorométhoxy.

X est un halogène choisi notament parmi l'iode, le brome ou le chlore, préférentiellement le chlore.

On peut citer comme exemples de 4-halogénonitrobenzènes le 2,4-dichloronitrobenzène, le 3,4 dichloronitrobenzène, le 2-fluoro-4-chloronitrobenzène, le 3-trifluoromethyl 4-chloronitrobenzène.

Le procéde de l'invention est particulièrement avantageux lors de l'utilisation des 4-chloronitrobenzènes, des dichloronitrobenzènes, un des chlores étant en position 4, et des 4-chloro trifluorométhylnitrobenzènes.

Les 4-halogènonitrobenzènes sont préparés selon des procédés bien connus de l'homme de l'art.

L'hydrogène utilisé dans le procédé de l'invention peut être introduit en une seule fois ou en continu, sous pression ou à la pression atmosphérique par bullage, sans que cette condition soit critique.

L'acide fluorhydrique utilisé comme solvant est de préférence, anhydre, sans que cela soit une condition critique, mais la présence d'eau peut entraîner une diminution de rendement. On utilise de préférence une quantité d'acide fluorhydrique telle que le rapport molaire HF au 4-halogénonitrobenzène soit compris entre 5 : 1 et 50 : 1.

On etend par catalyseur à base d'un métal le catalyseur sous forme métallique proprement dite aussi bien que sous forme d'oxyde, notamment le dioxide de platine, ou encore un mélange des deux, employé en tant que tel ou déposé sur un support du type charbon, silice, alumine, ou tout autre support bien connu de l'homme de l'art.

La quantité préférée de catalyseur est de 1 à 5% en poids par rapport au 4-halogénonitrobenzène.

Dans une mise en œuvre préférentielle, on utilisera comme catalyseur le paladium sur charbon, avantageusement dans un rapport palladium/charbon de 1 à 5% poids.

La pression utilisée est généralement comprise entre 10 et 20 bars, sans que cette condition soit critique.

Le procédé selon l'invention est de préférence mis en œuvre à une temperature comprise entre 20 et 250°C, plus préférentiellement entre 70 et 150°C.

La durée de réaction, généralement comprise entre 1 heure et 10 heures varie avec le produit de départ ainsi qu'avec la température et la presson employées. L'homme du métier saura adapter ces paramètres à l'économie du procéde.

Parmi les 4-fluoroanilines obtenues par le procédé selon l'invention on trouvera notamment les composés de formule générale

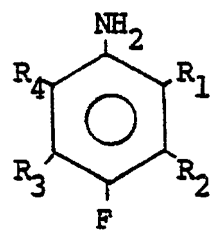

ou $R_1$, $R_2$, $R_3$ et $R_4$ correspondent à la définition donnée précédemment.

Selon une variante du procédé de la présente invention on peut utiliser en outre tout composé augmentant l'acidité du milieu réactionnel notamment des acides de Lewis tels que $BF_3$, dont l'effet positif peut être une augmentation du rendement.

Le procédé selon l'invention se réalise en une seule étape avec une utilisation minimale de catalyseur. Ce dernier n'étant pas consommé, est aisément récupérable et recyclable. En outre par comparaison avec l'art antérieur, on peut éviter l'utilisation de catalyseurs toxiques et de manipulation délicate. Tout en étant un procédé simple n'employant pas de conditions sévères de pression et température, il donne de hauts rendements grâce à son excellente sélectivité.

Les 4-fluoroanilines sont des produits utiles comme intermédiaires dans la fabrication, de pesticides notamment de fongicides, d'herbicides et de produits pharmaceutiques.

Les exemples suivants sont donnés à titre indicatif et ne douvent pas être considérés comme une limite du domaine et de l'esprit de l'invention.

<u>EXEMPLE 1.</u> - Réduction du dichloro-2,4 nitrobenzène.

Dans un réacteur inox de 250 ml agité par barreau aimanté, on introduit sucessivement 100 g (5m) d'acide fluorhydrique anhydre, 19,2 g (0,1 m) de dichloro-2,4 nitrobenzène et 1 g de catalyseur Pd/C à 5 %. Le réacteur est fermé et purgé à l'azote. Ce mélange est chauffé par l'intermédiaire d'un bain d'huile à la température de 80°C, le chauffage est stoppée puis on introduit sous agitation de l'hydrogène gazeux de manière à obtenir un pression de 20 bars dans le réacteur et à un débit tel que la température se maintienne aux environ de 80°C. Après 3 heures de réaction on n'observe plus d'absorption d'hydrogène. Le mélange réactionnel est refroidi vers 0°C, le réacteur décomprimé et son contenu coulé sur 200 g de glace pilée. Après filtration, le brut acide est neutralisé jusqu'à pH 11 par de la potasse à 20 % et extrait par 2 fois 100 cm3 de $CH_2$ $Cl_2$. Les phases organiques sont rassemblées, séchées et enfin le solvant est évaporé sous pression réduite. On récupère ainsi 9 g d'un mélange dont les analyses par chromatographie

en phase gazeuse, spectrométrie IR et de masse nous montrent qu'il est constitué de

- 28,3 % de fluoro-4 chloro-2 aniline
- 20,8 % de dichloro-2,4 aniline
- 44,6 % de dichloro-2,4 nitrobenzène.

EXEMPLE 2. - Réduction du chloro-4 nitrobenzène.

On procède de manière analogue à l'exemple 1 avec les conditions et quantités suivantes :
- HF anhydre : 50 g - 2,5 moles
- Chloro-4 nitrobenzène : 31,6 g - 0,2 mole
- Catalyseur Pd/C à 5 % : 1 g
- Pression $H_2$ : 15 bars
- Température : 100°C
- Durée : 5 heures

Après traitements et analyses on récupère 20 g d'un mélange constitué de :
- fluoro-4 aniline : 48,4 %
- chloro-4 aniline : 9,9 %
- chloro-4 nitrobenzène : 28,7 %

EXEMPLE 3. - Réduction du bromo-4 nitrobenzène.

On procède de manière analoque à l'exemple 1 avec les conditions et quantités suivantes :
- HF anhydre : 100 g - 5 moles
- bromo-4 nitrobenzène : 60 g - 0,3 mole
- Catalyseur Pd/C à 1 % : 3 g
- Pression $H_2$ : 10 bars
- Température : 150°C
- Durée : 6 heures

Après traitements et analyses on récupère 45 g d'un mélange constitué de :
- Fluoro-4 aniline : 18 %
- Bromo-4 aniline : 35,6 %
- Bromo-4 nitrobenzène : 44 %

EXEMPLE 4. - Réduction du dichloro-3,4 nitrobenzène.

On procède de manière analoque à l'exemple 1 avec les conditions et quantités suivantes :
- HF anhydre : 100 g - 5 moles
- Dichloro-3,4 nitrobenzène : 76,8 g - 0,4 mole
- Catalyseur Pd/C à 2,5 % : 3 g
- Pression $H_2$ : 20 bars
- Température : 100°C
- Durée : 3 heures

Après traitements et analyses on récupère 50 g d'un mélange constitué de :
- fluoro-4 chloro-3 aniline : 38,9 %
- dichloro-3,4 aniline : 21,6 %
- dichloro-3,4 nitrobenzène : 32,1 %

EXEMPLE 5. - Réduction du chloro-4 trifluorométhyl-3 nitrobenzène.

On procède de manière analoque à l'exemple 1 avec les conditions et quantités suivantes :
- HF anhydre : 60 g - 3 moles
- Chloro-4 trifluorométhyl-3 nitrobenzène : 76,8 g - 0,4 mole
- Catalyseur Pd/C - 5 % : 2,5 g
- Pression $H_2$ : 20 bars
- Température : 80°C
- Durée : 7 heures

Après traitements et analyses on récupère 38 g d'un mélange constitué de :
- fluoro-4 trifluorométhyl-3 aniline : 53,5 %
- chloro-4 trifluorométhyl-3 aniline : 19,4 %
- chloro-4 trifluorométhyl-3 nitrobenzène : 25,2 %

EXEMPLE 6. - Réduction du chloro-4 méthyl-3 nitrobenzène.

On procède de manière analoque à l'exemple 1 avec les conditions et quantités suivantes :
- HF anhydre : 100 g - 5 moles
- Chloro-4 méthyl-3 nitrobenzène : 76,8 g - 0,4 mole
- Catalyseur Pd/C - 5 % : 1 g
- Pression $H_2$ : 15 bars
- Température : 80°C
- Durée : 2 heures

Après traitements et analyses on récupère 24 g d'un mélange constitué de :
- fluoro-4 méthyl-3 aniline : 46,2 %
- chloro-4 méthyl-3 aniline : 19,4 %
- chloro-4 méthyl-3 nitrobenzène : 31,7 %

EXEMPLE 7. - Réduction du chloro-4 trifluorométhoxy-3 nitrobenzène.

On procède de manière analoque à l'exemple 1 avec les conditions et quantités suivantes :
- HF anhydre : 80 g - 4 moles
- Chloro-4 trifluorométhoxy-3 nitrobenzène : 24 g - 0,1 mole
- Catalyseur Pd/C - 5 % : 2 g
- Pression $H_2$ : 20 bars
- Température : 100°C
- Durée : 5 heures

Après traitements et analyses on récupère 18 g d'un composé constitué de :
- fluoro-4 trifluorométhoxy-3 aniline : 47,2 %
- chloro-4 trifluorométhoxy-3 aniline : 12,7 %
- chloro-4 trifluorométhoxy-3 nitrobenzène : 31,7 %

**Revendications**

1. Procédé de préparation de 4-fluoroanilines à partir de 4-halogénonitrobenzènes dont l'halogéne en position de n'est pas le fluor caractérisé en ce que l'on fait réagir au moins un 4-halogénonitrobenzène dont l'halogéne en position n'est pas le fluor avec de l'hydrogene dans de l'acide fluorhydrique solvant, en présence d'au moins un catalyseur à base d'un métal choisi parmi le rhuténium, le rhodium, le palladium, l'osmium, l'irridium et le platine.

2. Procédé selon la revendication 1, caractérisé en ce que les 4-halogénonitrobenzènes ont la formule générale:

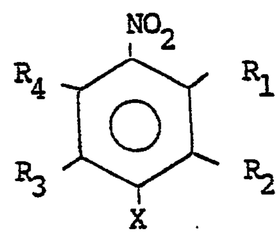

où $R_1$, $R_2$, $R_3$, et $R_4$ sont des substituants identiques ou différents représentant soit l'hydrogène soit des radicaux inertes dans les conditions réactionnelles;
X est un halogène choisi parmi le chlore, le brome et l'iode.

3. Procédé selon la revendication 2, caractérisé en ce que $R_1$, $R_2$, $R_3$ et $R_4$ sont choisis parmi les radicaux hydrogène, halogéno, alkyle, perhalogènoalkyle et perhalogènoalcoxy, les groupes alkyle et alcoxy ayant de 1 à 4 atomes de carbone.

4. Procédé selon l'une quelconque des revendications 2 ou 3, caractérisé en ce que le radical perhalogènoalkyle est un groupe perfluoroalkyle et le radical perhalogènoalcoxy est un groupe perfluoroalcoxy.

5. Procédé selon les revendications 2 à 4, caractérisé en ce que le radical perhalogènoalkyle est le radical trifluorométhyle, le radical perhalogènoalcoxy est le radical trifluorométhoxy, et X est le chlore.

6. Procéde selon l'une quelconque des revendications 1 à 5, caractérisé en ce que l'acide fluorhydrique est anhydre.

7. Procédé selon l'une quelconque des revendications 1 à 6, caractérisé en ce que le rapport molaire acide fluorhydrique au 4-halogénonitrobenzène est compris entre 5/1 et 50/1.

8. Procédé selon l'une quelconque des revendications 1 à 7, caractérisé en ce que le catalyseur est à base de palladium déposé sur charbon utilisé selon un rapport pondéral palladium/charbon compris entre 1 à 5%.

9. Procédé selon l'une quelconque des revendications 1 à 8 caractérisé en ce que la quantité de catalyseur constitue du metal ou d'un de ses oxydes déposé sur un support est comprise entre 1 et 5% en poids par rapport à la quantité de 4-halogénonitrobenzène.

10. Procédé selon l'une quelconque des revendications 1 à 9, caractérisé en ce que la température de réaction est comprise entre 20 et 250°C, préférentiellement entre 70 et 150°C.

11. Procédé selnon l'une quelconque des revendications 1 à 10 caractérisé en ce que la pression de réactionnelle est comprise entre 10 et 20 bars.

## Claims

1. Process for the preparation of 4-fluoroanilines from 4-halonitrobenzenes in which the halogen in the 4-position is not fluorine, characterized in that at least one 4-halonitrobenzene in which the halogen in the 4-position is not fluorine is reacted with hydrogen in solvent hydrofluoric acid, in the presence of at least one catalyst based on a metal chosen from amongst ruthenium, rhodium, palladium, osmium, iridium and platinum.

2. Process according to Claim 1, characterized in that the 4-halonitrobenzenes are of the general formula:

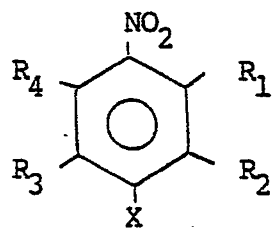

in which $R_1$, $R_2$, $R_3$ and $R_4$ are identical or different substituents which represent either a hydrogen atom or radicals which are inert under the conditions of the reaction; and X is a halogen atom chosen from amongst chlorine, bromine and iodine.

3. Process according to Claim 2, characterized in that $R_1$, $R_2$, $R_3$ and $R_4$ are chosen from amongst hydrogen, halo, alkyl, perhaloalkyl and perhaloalkoxy radicals, the alkyl and alkoxy groups containing 1 to 4 carbon atoms.

4. Process according to either of Claims 2 or 3, characterized in that the perhaloalkyl radical is a perfluoroalkyl group and the perhaloalkoxy radical is a perfluoroalkoxy group.

5. Process according to Claims 2 to 4, characterized in that the perhaloalkyl radical is the trifluoromethyl radical, the perhaloalkoxy radical is the trifluoromethoxy radical and X is chlorine.

6. Process according to any one of Claims 1 to 5, characterized in that the hydrofluoric acid is anhydrous.

7. Process according to any one of Claims 1 to 6, characterized in that the molar ratio of hydrofluoric acid to 4-halonitrobenzene is between 5:1 and 50:1.

8. Process according to any one of Claims 1 to 7, characterized in that the catalyst is based on palladium deposited on charcoal, used according to a palladium: charcoal ratio by weight of between 1 and 5%.

9. Process according to any one of Claims 1 to 8, characterized in that the quantity of catalyst, which consists of the metal or one of the oxides thereof deposited on a support, is between 1 and 5% by weight relative to the quantity of 4-halonitrobenzene.

10. Process according to any one of Claims 1 to 9, characterized in that the reaction temperature is between 20 and 250°C, preferably between 70 and 150°C.

11. Process according to any one of Claims 1 to 10, characterized in that the reaction pressure is between 10 and 20 bars.

## Patentansprüche

1. Verfahren zur Herstellung von 4-Fluoranilinen, ausgehend von 4-Halogennitrobenzolen, bei denen das Halogen in 4-Stellung nicht Fluor ist, dadurch gekennzeichnet, daß man mindestens ein 4-Halogennitrobenzol, bei dem das Halogen in 4-Stellung nicht Fluor ist, mit Wasserstoff in Fluorwasserstoffsäure als Lösungsmittel in Gegenwart mindestens eines Katalysators auf Basis eines Metalls, ausgewählt aus Ruthenium, Rhodium, Palladium, Osmium, Irridium und Platin, umsetzt.

2. Verfahren nach Anspruch 1, dadurch gekennzeichnet, daß die 4-Halogenitrobenzole die allgemeine Formel

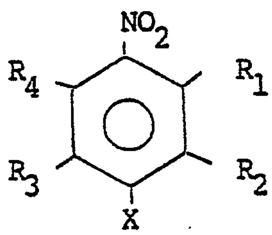

besitzen, in der $R_1$, $R_2$, $R_3$ und $R_4$ gleiche oder verschiedene Substituenten sind, die Wasserstoff oder unter den Rektionsbedingungen inerte Reste darstellen, und X ein Halogenatom ist, ausgewählt aus Chlor, Brom und lod.

3. Verfahren nach Anspruch 2, dadurch gekennzeichnet, daß $R_1$, $R_2$, $R_3$ und $R_4$ ausgewählt sind aus Wasserstoff-, Halogen- oder Alkyl-, Perhalogenalkyl- und Perhalogenalkoxygruppen, wobei die Alkyl- und Alkoxygruppen 1 bis 4 Kohlenstoffatome enthalten.

4. Verfahren nach einem der Ansprüche 2 oder 3, dadurch gekennzeichnet, daß der Perhalogenalkylrest eine Perfluoralkylgruppe und der Perhalogenalkoxyrest eine Perfluoralkoxygruppe ist.

5. Verfahren nach den Ansprüchen 2 bis 4, dadurch gekennzeichnet, daß der Perhalogenalkylrest der Trifluormethylrest, der Perhalogenalkoxyrest der Trifluormethoxyrest und X Chlor ist.

6. Verfahren nach einem der Ansprüche 1 bis 5, dadurch gekennzeichnet, daß die Fluorwasserstoffsäure wasserfrei ist.

7. Verfahren nach einem der Ansprüche 1 bis 6, dadurch gekennzeichnet, daß das Molverhältnis Fluorwasserstoffsäure zu 4-Halogennitrobenzol zwischen 5:1 und 50:1 liegt.

8. Verfahren nach einem der Ansprüche 1 bis 7, dadurch geKennzeichnet, daß der Katalysator auf Basis von Palladium auf Aktivkohle ist und in einem Gewichtsverhältnis Palladium zu Aktivkohle zwischen 1 und 5% verwendet wird.

9. Verfahren nach einem der Ansprüche 1 bis 8, dadurch gekennzeichnet, daß die Menge des Katalysators, bestehend aus Metall oder einem seiner Oxide, abgeschieden auf einem Träger, zwischen 1 und 5 Gew.-%, bezogen auf die Menge an 4-Halogennitrobenzol ist.

10. Verfahren nach einem der Ansprüche 1 bis 9, dadurch gekennzeichnet, daß die Reaktionstemperatur zwischen 20 und 250°C, vorzugsweise zwischen 70 und 150°C, liegt.

11. Verfahren nach einem der Ansprüche 1 bis 10, dadurch gekennzeichnet, daß der Reaktionsdruck zwischen 10 und 20 bar beträgt.